Europäisches Patentamt

European Patent Office

Office européen des brevets

(19)

(11) EP 0 394 938 B1

(12) **EUROPEAN PATENT SPECIFICATION**

(45) Date of publication and mention
of the grant of the patent:
**31.01.1996 Bulletin 1996/05**

(51) Int Cl.$^6$: **C07H 17/08**

(21) Application number: **90107692.7**

(22) Date of filing: **24.04.1990**

(54) **Process for producing partricin or its methyl ester**

Verfahren zur Herstellung von Partricin oder dessen Methylester

Procédé de préparation de partricin ou son ester de méthyle

(84) Designated Contracting States:
**BE DE FR GB IT**

(30) Priority: **26.04.1989 JP 106167/89**
**07.09.1989 JP 230357/89**
**07.03.1990 JP 53807/90**

(43) Date of publication of application:
**31.10.1990 Bulletin 1990/44**

(73) Proprietor: **AJINOMOTO CO., INC.**
**Tokyo 104 (JP)**

(72) Inventors:
• **Ishi, Koichi, c/o Central Research Lab.,**
**Ajinomoto**
**Kawasaki-shi, Kanagawa-ken (JP)**

• **Miyashiro, Shigeyoshi,**
**c/o Central Research Lab.**
**Kawasaki-ku, Kawasaki-shi, Kanagawa-ken**
**(JP)**

(74) Representative:
**Strehl Schübel-Hopf Groening & Partner**
**D-80538 München (DE)**

(56) References cited:
**US-A- 4 642 318**

• **CANADIAN JOURNAL OF CHEMISTRY, vol. 63,**
**no. 1, January 1985, pages 77-85; N.O.**
**PETERSEN: "Intramolecular fluorescence**
**energy transfer in nitrobenzoxadiazole**
**derivatives of polyene antibodies"**

## Description

The present invention relates to a process for efficiently producing trans-type partricin or its methyl ester by irradiation with a radiation containing ultraviolet rays. The substance obtained by the process of the present invention has antifungal activity and can thus be used as a medical drug, a drug for animal treatment, etc.

Partricin is a known polyene antibiotic having a cis-type polyene structure (J. Antibiotics, 35, 997 (1982)). Whether the steric structure of such polyenes is trans-type or cis-type is already determined during the stage in which these antibiotics are produced by microorganisms in fermentation solutions, and at present, it is extremely difficult to artificially change this steric structure. It is known, however, that by exposing to light a diluted solution of partricin which is a cis-type polyene antibiotic, the maximum absorption wavelength in the ultraviolet region is shifted by 4 nm to the longer wavelength region. It is surmised that this wavelength shift occurs because cis-type partricin will be transformed to trans-type (US-A-3 773 925).

In general, polyenes are very unstable when exposed to light. For example, as described in the above stated literature, even when a partricin solution is simply exposed to light, conversion into trans-type occurs and at the same time, the formed trans-type compound is decomposed so that the product is finally completely decomposed. There is no case known, in which the trans-type compound obtained by the photoreaction could be isolated as a chemically pure substance. Further, no process could be found, which was utilized as an industrially applicable process for the production of the trans-compound by light exposure. It has therefore been desired to develop a process for efficiently converting cis-type polyene antibiotics into trans-type polyene antibiotics in a simple manner and without causing decomposition of the compound.

As a result of extensive investigations to solve the foregoing problem, the present inventor has found a process for efficiently converting cis-type partricin into the corresponding trans-type polyene antibiotic by irradiating a solution of the cis-type polyene antibiotic with ultraviolet rays in the long wavelength region and has come to accomplish the present invention, based on this finding.

The present invention relates to a process for producing partricin or its methyl ester having a trans-type polyene structure, which comprises irradiating a solution of the corresponding polyene antibiotic having a cis-type structure with radiation containing ultraviolet rays of the longer wavelength region.

As polyene antibiotics having a cis-type polyene structure, there are known partricin (J. Antibiotics, 35, (8), 997-1012, (1982), U.S. Patent No. 3,773,925), partricin methyl ester (Japanese Patent Publication No. 55-50960), etc. These antibiotics may be purified products or may be in the state of crude products which contain impurities when used for the purpose of this invention.

Solvents for dissolving partricin or its methyl ester are desirably hydrophilic organic solvents such as methanol, ethanol, acetonitrile, dimethylformamide, dimethylacetamide, dimethylsulfoxide, etc.; and water-containing organic solvents containing at least 30% of these organic solvents. An alcohol aqueous solution containing approximately 5 to 30% of water, especially aqueous methanol solution, is preferred. It is more preferred to add salts to the solution. The salt is preferably an alkali or ammonium salt or an alkaline earth metal salt and may preferably be sodium chloride, sodium acetate, ammonium acetate, etc. but the kind of the salt is not limited. The concentration of the salt may be 5 mM or more, preferably 5 to 1000 mM, more preferably 10 mM to 200 mM. It is appropriate that the concentration of the polyene antibiotic in the solution be approximately 1 to 1000 mg/l. When the solvent is a water-containing solvent, it is preferred to adjust the pH to below 10, especially 5.5 to 9, in order to minimize the decomposition of the produced trans-type polyene antibiotics.

The wavelength of ultraviolet rays used in the present invention is 300 to 400 nm. Light or radiation in a longer wavelength region may also be used together with wavelengths of from 300 to 400 nm, since such a light does not cause isomerization. On the other hand, it is preferred not to use radiation in a wavelength region shorter than 300 nm, because such radiation causes decomposition of polyene antibiotics. The decomposition products are considered to be substances in which the polyene chromophore formed by cleavage of the polyene bond is ring-opened. The quantum efficiency by irradiation at about 300 nm is approximately $2 \times 10^{-2}$. The term quantum efficiency refers to the ratio of reacted molecules to the photons absorbed.

As a light source, a variety of light sources which can be applied industrially are used. For example, a natural light, a daylight fluorescent lamp, a cold white fluorescent light, a medium near-ultraviolet ray lamp, a long wavelength ultraviolet ray lamp, etc. may be used. Of these, a long wavelength ultraviolet ray lamp emitting a single ultraviolet radiation at 365 nm is particularly preferred. The time period for irradiation is determined in such way that the conversion into the trans-type is sufficiently achieved.

The purification of the trans-type compound can be performed by reverse phase chromatography which is a conventional method for isolation and purification of polyene antibiotics. For example, column chromatography, using Capcell pak ODS column (manufactured by Shiseido Co., Ltd.), LRP-1 (manufactured by Whatmann Co., Ltd., carrier for C-18 reverse phase chromatography), etc. is effective.

By irradiating a solution of a polyene antibiotic having a cis-type polyene structure with a radiation or light containing ultraviolet rays in the long wavelength region according to the present invention, the cis-type polyene antibiotic is excited and isomerized to the corresponding trans-type polyene antibiotic.

According to the process of the present invention, polyene antibiotics having a trans-type polyene structure which can be used as medical drugs or animal drugs can be prepared from polyene antibiotics having a cis-type polyene structure, which have a strong toxicity as medical drugs or animal drugs, in simple apparatuses and operations in a high yield, by converting the cis-type polyene antibiotics into the trans-type polyene antibiotics.

The invention is further illustrated by the enclosed drawings, in which

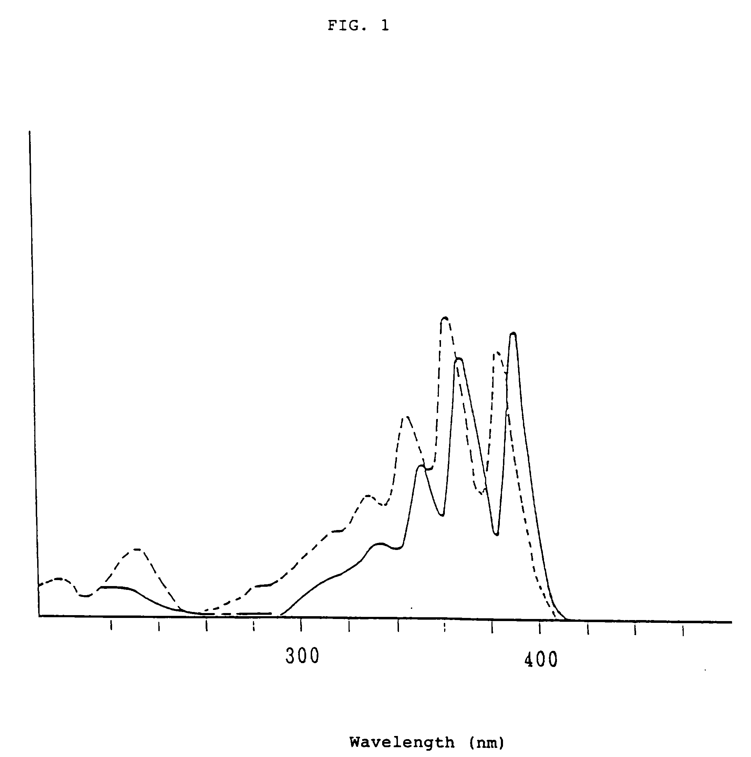

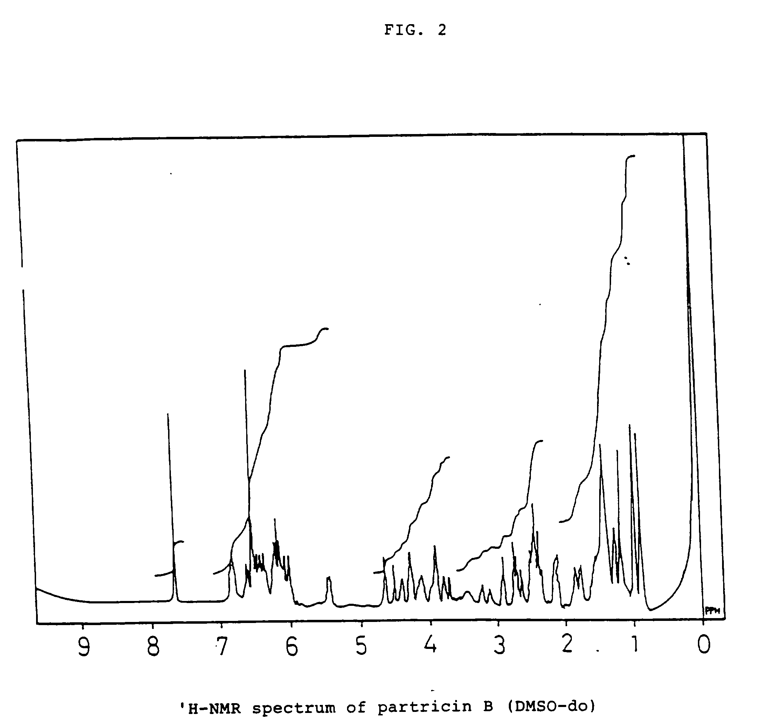

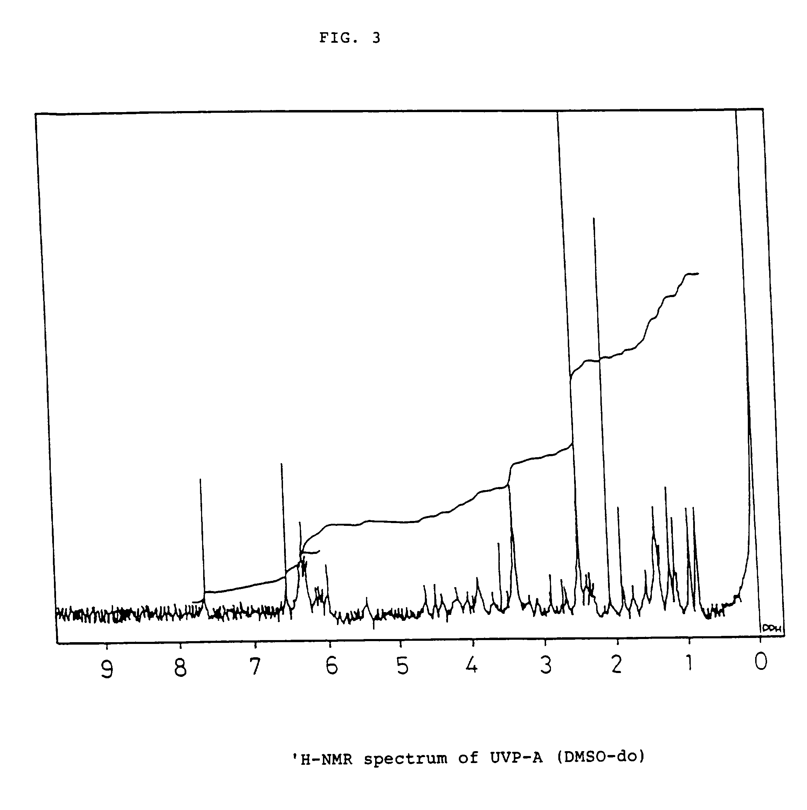

Fig. 1 is a polyene antibiotic having a trans-type polyene structure obtained by the process of the present invention, wherein UV spectrum (solid line) of the trans isomer of partricin B is shown by comparing with UV spectrum (broken line) of partricin B which is a polyene antibiotic having a cis-type polyene structure. Fig. 2 is a drawing showing $^1$H-NMR of partricin B. Fig. 3 is a drawing showing $^1$H-NMR of UVP-A.

Example 1

One kiloliter of medium having the composition shown in Table 1 was sterilized by heating and charged in a fermenter. Partricin B-producing Streptomyces arenae (FERN P-8099, FERM BP-1537) was inoculated into the medium followed by culturing at 28°C for 3 days.

Table 1

| Glucose | 6 % |
| Sodium glutamate | 4 % |
| Yeast extract | 0.2 % |
| Polypeptone | 0.2 % |
| Magnesium sulfate 7 hydrate | 0.01 % |
| Sodium chloride | 0.5 % |

After culturing, mycelia were collected by centrifuqation and 100 ℓ of methanol were added thereto. After pH was adjusted to 6.0 with acetic acid, the system was allowed to stand at room temperature for 24 hours. Then, filtration was performed and 100 ℓ of the resulting filtrate were concentrated to 10 ℓ under reduced pressure. The precipitates were collected by centrifugation and freeze dried to give 200 g of crude partricin B (purity 65%).

Crude partricin B (100 g) was purified by reverse phase partition HPLC (Capcell pak ODS column, manefactured by Shiseido Co., Ltd.) to give 80 g of purified dry partricin B (purity, 98%). The physicochemical properties of purified partricin B were examined and found to be identical with those of known partricin B (J. Antibiotics, 35, 997 (1982). The following parameters were determined : molecular weight of 1112 (determined by FABMS), Rf value of 0.34 in thin layer chromatogram [manufactured by Whatmann Co., Ltd., PLKC18F TLC plate, developing solvent: $CH_3CN$-50 mM $NH_4OAc$ (pH 9.0) = 45 : 55]; retention time of 5.10 minutes in HPLC [column: YMC AM-312 Ø 4.5 x 150 mm, solvent: $CH_3CN$-50 mM $NH_4OAc$ (pH 5.5) = 4.5 : 5.5, flow rate: 1.00 ml/min], UV absorption spectrum (measured in MeOH, shown by broken line in Fig. 1) and IR spectrum, giving a single spot by TLC and a single spot by HPLC.

The purified partricin B prepared by the above method was dissolved in 90% methanol solution (50 mM ammonium acetate was added and pH was adjusted to 5.8 with acetic acid) in a concentration cf 40 mg/ℓ. One liter of this solution was charged in a stainless vat in a solution depth of 1 cm in a dark room. While gently stirring, the solution was irradiated with two long wavelength ultraviolet ray lamps (wavelength of 365 nm, UV lamp of 8 W, intensity of 410 μw/cm$^2$) at a distance of 12.7 cm for 20 minutes. The irradiated solution was concentrated to 100 ml under reduced pressure and the formed precipitates were collected by centrifugation. The precipitates were washed twice with 25 ml of water and dissolved in a small quantity of tetrahydrofuran solution. The resulting solution was applied to LRP-1 reverse phase chromatography column, which was eluted with a mixture of acetonitrile : 0.05 ammonium acetate (pH 9.0) = 4.5 : 5.5. The absorbance was measured at 360 nm and eluted fractions of the product obtained by irradiation with ultraviolet rays (hereafter referred to as UVP-A) were collected. After concentrating under reduced pressure, the residue was allowed to stand in a refrigerator overnight. Next day, the precipitated yellow crystals of UVp-A were collected by centrifugation and dried to give 30 mg of purified UVP-A. The physicochemical properties of UVP-A are as follows.

(1) Elemental analysis:       C: 62.85%, H: 7.66%, N: 2.55%
(2) Molecular weight:       1112 (determined by FABMS)
(3) Optical rotation:       $[\alpha]_D^{26}$:+ 335° (C = 0.1% dimethyl sulfoxide)

(4) UV spectrum:       as shown in Fig. 1 (measured in MeOH, shown by solid line)
(5) Rf value :       0,26 (PLKC18F TLC plate, manufactured by Whatmann Co., Ltd., developing

solvent: $CH_3CN$-50 mM $NH_4OAc$ (pH 9.0) = 45 : 55]

(6) Retention time of HPLC:    8.00 minutes

[column: YMC AM-312 $\phi$ 4.5 x 150 mm, solvent: $CH_3CN$-50 mM $NH_4OAc$ (pH 5.5) = 4.5 : 5.5, flow rate: 1.00 ml/min]

A comparison of the physicochemical properties between partricin B and UVP-A is shown in Table 2. As shown in the table, the molecular weight is identical and UV spectrum and optical rotation are different, indicating that UVP-A. is a trans-type isomer of partricin B. When $^1$H-NMR data of both antibiotics (Fig. 2, Fig. 3) are compared, they are well identical as a whole. A difference is noted between 5.5 and 6.5 ppm but this indicates a difference in steric structure of the polyene structure in both compounds.

Table 2

| Physicochemical Property | Partricin B | UVP-A |
|---|---|---|
| Molecular weight | 1112 | 1112 |
| $UV\lambda_{max}^{MeOH}(\varepsilon)$ | 400 ( 87, 558 ) | 404 (150, 380 ) |
| | 378 (100, 425 ) | 380 (130, 700 ) |
| | 358 ( 73, 392 ) | 361 ( 82, 900 ) |
| $[\alpha]_D^{26}$ | + 114° | + 335° |

As shown in Table 3, UVP-A has a potent antibacterial activity as compared to partricin B. The minimum growth inhibitory concentration (MIC) against <u>Candida</u> <u>albicans</u> was 0.005 µg/ml.

Table 3

| Strain | MIC (µg/ml) | |
|---|---|---|
| | Partricin B | UVP-A |
| <u>Candida</u> <u>albicans</u> ATCC 10231 | 0.04 | 0.005 |
| <u>Saccharomyces</u> <u>ubran</u> ATCC 9080 | 0.04 | 0.005 |

Example 2

Purified partricin B was prepared in a manner similar to Example and dissolved in a concentration of 40 mg/$\ell$. One liter each of the solution was irradiated with radiation having various wavelengths. The produced UVP-A was isolated and its quantity was determined. The results are shown in Table 4.

Table 4

| Wavelength (nm) | Quantity of UVP-A Formed (mg) |
|---|---|
| 225 | 0 |
| 320 | 7 |
| 365 | 28 |
| 400 | 8 |
| 436 | 0 |
| 580 | 0 |

Example 3

Partricin B was irradiated with light using various light sources in a manner similar to Example 2. As the result, the formed UVP-A is as shown in Table 5.

Table 5

| Kind of Light Source | Quantity of UVP-A formed (mg) Time of Irradiation | |
|---|---|---|
| | 20 Minutes | 72 Hours |
| Natural light (sunlight) | 12 | * |
| Daylight fluorescent lamp | 0 | 12 |
| Cold white fluorescent lamp | 0 | 12 |
| Medium near-UV fluorescent lamp | 12 | * |
| Long wavelength UV lamp | 28 | * |

**\*not measured**

Example 4

One liter (containing 50 mg of partricin B) of the methanol extract of the cultured cells of <u>Streptomyces</u> <u>arenae</u> obtained in a manner similar to Example 1 was irradiated with ultraviolet rays in a manner similar to Example 1. UVP-A was isolated from the irradiated solution to give 20 mg of the purified product as a dry product. Example 5

One liter of 40 mg/$\ell$ partricin B solutions dissolved in various solvents in a manner similar to Example 1 and Example 2 was irradiated with two long wavelength ultraviolet ray lamps (wavelength of 365 nm, UV lamp of 8 W, intensity of 410 $\mu$w/cm$^2$) at a distance of 12.7 cm for 20 minutes. The produced UVP-A was isolated and its quantity was determined. The results are shown in Table 6.

Table 6

| Kind of Solvent | Quantity of UVP-A Produced (mg) |
|---|---|
| Water | < 5 |
| 90% Methanol aqueous solution | 28 |
| 80% Methanol aqueous solution | 26 |
| 90% Ethanol aqueous solution | 25 |
| Methanol | < 5 |
| Ethanol | < 5 |
| Acetonitrile | < 5 |
| Dimethylformamide | 8 |
| Dimethylacetamide | 8 |
| Dimethylsulfoxide | 8 |

Example 6

In 250 ml of dimethylsulfoxide was dissolved 30 g of partricin B prepared in a manner similar to Example 1. While slowly stirring at room temperature, 200 ml of ether solution of diazomethane was added to the solution. Then, the mixture was allowed to stand overnight in such a state that light was shielded. Ether was added to the mixture to precipitate the product. The precipitates were recovered by filtration. After washing with ether, the precipitates were dried in vacuum. The resulting crude partricin B methyl ester was purified by the method described in Japanese Patent Publication No. 55-50960 to give 5 mg of the purified ester as a dry product. This compound showed the following physicochemical properties and was identified to be partricin B methyl ester (substance described in Japanese Patent Publication No. 55-50960).

(1) Property: yellow powder
(2) Molecular weight: 1126 (determined by FABMS)
(3) Molecular formula: $C_{59}H_{86}N_2O_{19}$
(4) UVmax: 401, 378, 359, 340nm

Furthermore, absorption at 1715 cm$^{-1}$ derived from C=O of the methyl ester was observed in IR absorption spectrum and the characteristic peak of the methyl group at 3.25 ppm due to the presence of ester group was noted in $^1$H-NMR.

Example 7

Crude partricin B methyl ester, 5 mg, obtained in a manner similar to Example 6 was dissolved in 1 liter of 90% MeOH solution (50 mM ammonium acetate was added and pH was adjusted to 6.0 with acetic acid). The solution was irradiated with ultraviclet rays of 365 nm, using a long wavelength ultraviolet ray lamp in a manner similar to Example 1. The irradiated solution was concentrated and the formed precipitates were recovered by centrifugation. Then the precipitates were purified by high performance liquid chromatography (HPLC, flow rate of 1.0 ml/min) using Novaback C18 (product by Waters Co., Ltd.) as a carrier, 10 mM ammonium acetate buffer (pH 8.5) containing 65% acetonitrile as a moving phase and UV (405 nm) detector as a detector. The peak eluted at 7.5 minutes was recovered and concentrated in vacuum. The formed precipitates were collected by centrifugation and dried. The dry product was obtained in an amount of 1.5 mg. The purified product (referred to as UVP-B) showed the following properties.

(1) Property: yellow powder
(2) Molecular weight: 1126 (determined by FABMS)
(3) Molecular formula: $C_{59}H_{86}N_2O_{19}$
(4) UVmax: 405, 382, 344nm

When the physicochemical properties of partricin B methyl ester are compared with those of UVP-B, the molecular weight and molecular formula are identical but the maximum wavelength of UV absorption is different, indicating that partricin B methyl ester having a cis-type polyene structure was converted to UVF-B having a trans-type polyene structure. Furthermore, the minimum growth inhibitory concentration (MIC) of UVP-B against Candida albicans ATCC 10231 was 0.39 μg/ml. UVP-B showed a more potent activity than partricin B methyl ester (MIC = 0.52).

Example 8

One liter of 95% ethanol aqueous solutions containing 40 mg/ℓ partricin B, to which 50 mM of various salts were added and whose pH had been adjusted to 8 in a manner similar to Example 1 and Example 2, was irradiated with two long wavelength ultraviolet ray lamps (wavelength of 365 nm, UV lamp of 8 W, intensity of 410 μW/Cm$^2$) at a distance of 12.7 cm for 60 minutes The produced UVP-A was isolated and its quantity was determined. The results are shown in Table 7.

Table 7

| Kind of Salt | Quantity of UVP-A produced (mg) |
|---|---|
| No addition | 0.4 |
| Ammonium acetate | 39 |
| Potassium acetate | 36 |
| Sodium acetate | 35 |
| Sodium chloride | 32 |
| Ammonium chloride | 32 |

Example 9

One milliliter of 95% ethanol aqueous solution of 20 mg/ℓ partricin B, to which 50 mM of ammonium acetate had been added and whose pH had been adjusted to 8 in a manner similar to Example 1 and Example 2, was irradiated with radiation having wavelengths of 302, 320, 265 and 405 nm for 30 minutes, using a fluorescent spectrophotometer. The amount of the produced UVP-A was quantitatively determined. The quantitative determination was performed by reverse phase column YMC pack AM-312 (manufactured by Yamamura Chemical Research Institute), using as an eluting solution acetonitrile : 50 mM ammonium acetate (pH 5.5) = 4.5 : 5.5, whereby the quantum efficiency was determined. The results are shown in Table 8. The irradiation described above was carried out under such conditions that partricin B was converted into UVP-A by 100% and the produced UVF-A was not decomposed.

Table 8

| Wavelength | Quantum Efficiency |
|---|---|
| 302nm | $2.576 \times 10^{-2}$ |
| 320nm | $1.692 \times 10^{-2}$ |
| 365nm | $3.809 \times 10^{-3}$ |
| 405nm | $2.024 \times 10^{-3}$ |

## Claims

1. A process for producing partricin or its methyl ester having a trans-type polyene structure, which comprises irradiating a solution of partricin or its methyl ester having a cis-type structure with radiation containing ultraviolet rays of the wavelength regions of 300 nm to 400 nm.

2. A process according to claim 1, wherein said solution of the cis-type partricin or its methyl ester comprises a salt.

3. A process according to claim 2, wherein the salt concentration of said solution is at least 5 mM.

4. A process according to any of the claims 1 to 3, wherein the solvent of said solution is a hydrophilic organic solvent.

## Patentansprüche

1. Verfahren zur Herstellung von Partricin oder dessen Methylester, die eine Polyenstruktur des trans-Typs aufweisen, das darin besteht, daß eine Lösung von Partricin oder dessen Methylester, die eine Struktur des cis-Typs aufweisen, mit Strahlung, die Ultraviolettstrahlen des Wellenlängenbereiches von 300 nm bis 400 nm enthält, bestrahlt wird.

2. Verfahren nach Anspruch 1, wobei die Lösung von cis-Partricin oder dessen Methylester ein Salz enthält.

3. Verfahren nach Anspruch 2, wobei die Konzentration des Salzes in der Lösung mindestens 5 mM ist.

4. Verfahren nach einem der Ansprüche 1 bis 3, wobei das Lösungsmittel dieser Lösung ein hydrophiles organisches Lösungsmittel ist.

## Revendications

1. Procédé de production de partricine ou de son ester méthylique ayant une structure polyéne de type trans, qui comprend d'irradier une solution de partricine ou de son ester méthylique ayant une structure de type cis avec une radiation contenant des rayons ultraviolets dans la région de longueur d'onde 300 nm à 400 nm.

2. Procédé selon la revendication 1, où ladite solution de partricine de type cis ou de son ester méthylique comprend un sel.

3. Procédé selon la revendication 2, où la concentration du sel de ladite solution est au moins 5 mM.

4. Procédé selon l'une quelconque des revendications 1 à 3, où le solvant de ladite solution est un solvant organique hydrophile.

FIG. 1

Wavelength (nm)

FIG. 2

'H-NMR spectrum of partricin B (DMSO-do)

FIG. 3

'H-NMR spectrum of UVP-A (DMSO-do)